# FASCICULE DE BREVET EUROPEEN

(11) **EP 0 694 312 B1**
(45) Date de publication et mention de la délivrance du brevet: **10.12.2003**
(21) Numéro de dépôt: 95420216.4
(22) Date de dépôt: 24.07.1995
(51) Int. Cl.: A61M 1/16

(54) **Dispositif et procédé pour préparer un liquide de traitement par filtration**
Vorrichtung und Verfahren zur Vorbereitung einer Behandlungsflüssigkeit durch Filtrierung
Device and process for preparing a treatment fluid by filtration

(30) Priorité: 26.07.1994 FR 9409499
(43) Date de publication de la demande: 31.01.1996
(60) Demande divisionnaire: 02080290.6
(73) Titulaire: HOSPAL INDUSTRIE, F-69883 Meyzieu Cédex (FR)
(72) Inventeur: Riquier, Jean-Claude, F-69140 Rillieux (FR); Chevallet, Jacques, F-69360 Serezin du Rhone (FR)
(74) Mandataire: Sutto, Luca

(56) Documents cités:
- EP-A- 0 121 085
- EP-A- 0 570 349
- EP-A- 0 571 303
- US-A- 4 244 820
- US-A- 5 066 402

## Description

La présente invention a pour objet un dispositif de traitement de sang par circulation extracorporelle équipé pour produire un liquide de traitement stérile et apyrogène.

Les patients souffrant d'insuffisance rénale peuvent être soumis à différents traitements impliquant une circulation extracorporelle de sang, l'hémodialyse, l'hémofiltration et l'hémodiafiltration.

L'hémodialyse consiste à faire circuler le sang du patient dans un premier compartiment d'un dialyseur et un liquide de dialyse dans un second compartiment du dialyseur, les deux compartiments étant séparés par une membrane semi-perméable permettant un transfert diffusif de solutés au travers de la membrane, du compartiment où la concentration en un soluté particulier est la plus importante vers le compartiment où la concentration en ce soluté est la moins importante.

L'hémofiltration consiste à extraire du sang du patient une fraction d'eau plasmatique ou filtrat au moyen d'un hémofiltre et à infuser simultanément au patient un liquide de substitution pour compenser, en général seulement partiellement, la quantité de filtrat extraite.

L'hémodiafiltration est une combinaison des deux traitements précités.

Le liquide de dialyse et le liquide de substitution sont des liquides ayant sensiblement la même composition. lls sont isotoniques au sang dont ils contiennent les principaux électrolytes.

Dans les machines de dialyse classiques, le liquide de dialyse est préparé par mélange d'eau et de solutions concentrées ou de sels pulvérulents, comprenant les principaux électrolytes du sang. Ce liquide de dialyse n'est ni stérile ni apyrogène, c'est-à-dire qu'il peut contenir des micro-organismes vivants (bactéries) ainsi que des éléments, dits pyrogènes, dont l'introduction dans le corps peuvent produire des effets indésirables, tels que fièvre, frissons, nausées ou réactions anaphylactoïdes.

Bien que la membrane du dialyseur isole le sang du liquide de dialyse et que, en cours de traitement, une pression transmembranaire positive soit établie entre le compartiment sang et le compartiment liquide de dialyse du dialyseur pour prévenir le passage de liquide de dialyse dans le sang, toute contamination du sang par des bactéries et des éléments pyrogènes contenus dans le liquide de dialyse n'est pas totalement exclue, notamment dans le cas d'une rupture de filtre ou dans le cas d'une inversion accidentelle du sens de la pression transmembranaire lorsqu'un dialyseur à haute perméabilité hydraulique est utilisé.

Aussi l'intérêt d'utiliser du liquide de dialyse stérile et apyrogène a été exprimé à plusieurs reprises. On a en particulier proposé de filtrer le liquide de dialyse dans un filtre ayant une première et une seconde chambre séparées par une membrane ayant une perméabilité hydraulique élevée, la première chambre ayant une entrée pour l'introduction du liquide à filtrer et une sortie pour l'évacuation des substances retenues par la membrane avec une fraction du liquide introduit dans le filtre (voir "Investigation of the Permeability of Highly Permeable Polysulfone Membranes for Pyrogens" In Contr. Nephrol., vol. 46, pp. 174-183, Karger, Basel 1985).

Le brevet européen 0 270 794 décrit une machine de dialyse dont le circuit de liquide de dialyse comprend :
- une canalisation d'alimentation sur laquelle est disposé un filtre ayant une première et une seconde chambres séparées par une membrane de filtration, la canalisation d'alimentation ayant une première portion reliant une source de liquide de dialyse à une entrée de la première chambre, et une seconde portion ayant une extrémité connectée à une sortie de la seconde chambre du filtre et une autre extrémité connectable à une entrée d'un premier compartiment d'un dialyseur ;
- une canalisation d'évacuation ayant une extrémité connectable à une sortie du premier compartiment du dialyseur ; et
- une canalisation de purge, sur laquelle est disposée une vanne, reliant une sortie de la première chambre du filtre à la canalisation d'évacuation.

En fonctionnement, la vanne disposée sur la canalisation de purge est ouverte à intervalles de temps réguliers pour purger la première chambre du filtre des micro-organismes et éléments pyrogènes retenus par la membrane.

Cette machine présente plusieurs inconvénients. En particulier, il est clair que la vanne de la canalisation de purge ne peut être ouverte fréquemment à cause de la perturbation du traitement qui en résulte. (En effet; compte tenu de la perte de charge importante que représente la membrane du filtre, lorsque la vanne est ouverte, tout le liquide de dialyse provenant de la source de liquide de dialyse s'écoule par la canalisation de purge et le dialyseur n'est plus alimenté en liquide de dialyse frais). Par ailleurs, entre deux ouvertures successives de la vanne, il s'écoule un laps de temps pendant lequel les substances indésirables s'accumulent dans la première chambre du filtre où elles ont tendance à être entraînées par convection vers la membrane et à l'encrasser. Il en résulte que, en cas de rupture de la membrane du filtre, ces substances accumulées sont envoyées dans le dialyseur et la portion de circuit de dialyse située en amont et en aval de celui-ci, qu'elles contaminent. Aussi, le balayage tangentiel de la membrane qui résulte de l'ouverture intermittente de la vanne ne peut pas être suffisamment long pour décoller de la membrane toutes les substances qui y ont adhéré.

Le document EP 0 571 303 décrit un circuit hydraulique pour rein artificiel comprenant :
- un hémodialyseur ayant un premier et un second compartiments séparés par une membrane ;
- un ultrafiltre ayant une première et une seconde chambres séparées par une membrane ;
- une canalisation d'alimentation ayant un premier tronçon reliant une source de liquide de dialyse à une entrée de la première chambre de l'ultrafiltre et un second tronçon reliant une sortie de la seconde chambre de l'ultrafiltre à une entrée du premier compartiment de l'hémodialyseur ;
- une canalisation d'évacuation ayant une extrémité connectée à une sortie du premier compartiment de l'hémodialyseur ;
- une canalisation de purge connectée à une sortie de la première chambre de l'ultrafiltre.

Le document US 5 066 402 décrit un procédé pour sélectionner automatiquement, en fonction de valeurs de référence prédéterminées pour un paramètre de fonctionnement, l'un des trois modes de fonctionnement d'une installation de filtration d'eau, à savoir : un premier mode de fonctionnement où toute l'eau qui pénètre dans la première chambre du filtre utilisé passe au travers de la membrane du filtre ; un deuxième mode de fonctionnement où une partie de l'eau qui pénètre dans la première chambre du filtre est mise en recirculation dans cette chambre au moyen d'une canalisation bouclant une entrée et une sortie de la première chambre ; un troisième mode de fonctionnement où une partie de l'eau qui pénètre dans la première chambre du filtre est mise en recirculation dans cette chambre au moyen d'une canalisation bouclant une entrée et une sortie de la première chambre, et où une partie de l'eau mise en recirculation est purgée en permanence. Dans les trois mode de fonctionnement, la membrane est nettoyée par rétrofiltration, c'est-à-dire par passage forcé d'eau filtrée au travers de la membrane, du second compartiment du filtre vers le premier compartiment.

Un but de l'invention est de réaliser une machine de dialyse/hémofiltration capable de produire un liquide de traitement (liquide de dialyse, liquide de substitution) rendu stérile et apyrogène par filtration, dans laquelle le nettoyage du filtre utilisé ne provoque pas ou peu de perturbations au traitement en cours et permette d'optimiser la durée d'utilisation du filtre.

Pour atteindre ce but, on prévoit, selon l'invention, un dispositif de traitement de sang par circulation extracorporelle selon la revendication 1.

Grâce à cette disposition, le nettoyage de la membrane par balayage tangentiel est optimal, d'une part parce que ce balayage est continu et d'autre part parce que, étant indépendant du débit de circulation du liquide de dialyse dans le circuit de liquide de dialyse, il peut être ajusté (par réglage de la pompe de balayage) de façon à être le plus efficace possible. La durée d'utilisation du filtre est directement liée à l'efficacité du nettoyage auquel il est soumis.

Selon une caractéristique de l'invention, le dispositif comprend des moyens pour détecter un encrassement de la membrane du filtre et des moyens de commande pour commander l'organe de contrôle du débit dans la canalisation de purge en fonction d'un seuil d'encrassement prédéterminé de la membrane de façon à provoquer une purge de la première chambre du filtre.

Grâce à cette disposition, la fréquence de purge de la première chambre du filtre ne dépend que de la teneur du liquide de dialyse à filtrer en bactéries et en éléments pyrogènes de sorte que si l'eau utilisée pour la préparation du liquide de dialyse est très propre, la vanne de la canalisation de purge peut ne pas être ouverte ou seulement une ou deux fois par séance de traitement.

Selon une autre caractéristique de l'invention, le dispositif comprend des moyens pour calculer la fréquence des purges et la comparer la fréquence calculée à une fréquence de référence ; les moyens de commande sont alors prévus pour augmenter le débit de la pompe de balayage lorsque la fréquence des purges calculée atteint la fréquence de référence.

De la sorte, l'intensité du nettoyage du filtre peut être ajustée en fonction de la vitesse avec laquelle celui-ci s'encrasse, c'est-à-dire aussi en fonction du degré de pureté du liquide de dialyse provenant de la source de liquide de dialyse.

Selon une encore autre caractéristique de l'invention, le dispositif comporte des moyens pour mémoriser une pression transmembranaire initiale à la première mise en service du filtre et des moyens pour comparer la pression transmembranaire initiale à la pression transmembranaire mesurée. Le dispositif peut comporter en outre des moyens d'alarme pour émettre une alarme lorsque la pression transmembranaire mesurée s'écarte d'une quantité prédéterminée de la pression transmembranaire initiale.

On a observé que, à débit de liquide de dialyse égal dans le circuit de liquide de dialyse, la pression transmembranaire dans le filtre augmentait au cours du temps, de sorte que la comparaison entre le pression transmembranaire à la mise en service du filtre et la pression transmembranaire mesurée à un instant donné de l'utilisation du filtre donne une idée juste du vieillissement du filtre. On peut donc définir pour chaque filtre une durée d'utilisation individuelle optimale correspondant à une augmentation relative de la pression transmembranaire.

Selon encore une autre caractéristique de l'invention, le dispositif comporte des moyens de contrôle d'une quantité de liquide de dialyse, tels qu'un débitmètre, disposés sur la canalisation d'alimentation et le filtre est placé sur la canalisation d'alimentation entre la source de liquide de dialyse et les moyens de contrôle d'une quantité de liquide de dialyse.

Cette disposition présente plusieurs avantages : en premier lieu, les moyens de contrôle sont parcourus par un liquide filtré, ce qui est tout à fait approprié lorsque ces moyens sont constitués par un débitmètre, et particulièrement un débitmètre à turbine ou à engrenages dont le fonctionnement peut être sérieusement perturbé par une impureté solide et est en général altéré par un encrassement progressif. Par ailleurs, de tels moyens de contrôle peuvent faire partie d'un système de maîtrise volumétrique de l'ultrafiltration au moyen duquel, dans une portion du circuit de liquide de dialyse comprenant l'échangeur, la quantité de liquide de dialyse qui sort de cette portion de circuit est maintenue égale à la quantité de liquide de dialyse qui y entre. De seconds moyens de contrôle de la quantité de liquide de dialyse sont alors disposés sur la canalisation d'évacuation du circuit de liquide de dialyse. En connectant le filtre au circuit de liquide de dialyse à l'extérieur de la portion du circuit de liquide de dialyse où circule un volume constant de liquide, on évite d'avoir à connecter la canalisation de purge à la canalisation d'évacuation du circuit de dialyse en amont des seconds moyens de contrôle, c'est-à-dire aussi d'avoir à y introduire les impuretés retenues par le filtre, comme c'est le cas dans le dispositif décrit dans le brevet européen 0 270 794 mentionné plus haut. Encore un autre avantage de cette disposition est que la canalisation de purge peut être reliée directement à l'égout par une voie de sortie de la machine de dialyse indépendante de la voie de sortie par laquelle la canalisation d'évacuation de liquide usé est reliée à l'égout. De la sorte, la portion du circuit de liquide de dialyse qui se trouve en amont du filtre est totalement isolée, par le filtre, de la portion de circuit de liquide de dialyse qui se trouve en aval du filtre. La contamination de la canalisation d'évacuation, en aval comme en amont (rétro-contamination) de la jonction de la canalisation de purge à la canalisation d'évacuation, qui se produit sur le dispositif décrit dans l'état de la technique, est donc totalement évitée.

L'invention a également pour objet un procédé de nettoyage selon la revendication 14.

Selon une caractéristique de l'invention, le procédé comprend en outre les étapes de :
- mesurer un niveau d'encrassement du filtre ;
- comparer le niveau d'encrassement mesuré à un seuil d'encrassement prédéterminé ; et
- purger la première chambre du filtre lorsque le niveau d'encrassement mesuré atteint le niveau d'encrassement prédéterminé.

Selon une autre caractéristique de l'invention, le procédé consiste en outre à comparer la fréquence effective des purges de la première chambre du filtre à une fréquence de référence et à augmenter le débit de recirculation dans la première chambre du filtre lorsque la fréquence effective atteint la fréquence de référence.

Le procédé peut être particulièrement adapté à un filtre disposé dans un système de maîtrise volumétrique d'ultrafiltration d'une machine d'hémodialyse/hémofiltration, le système de maîtrise d'ultrafiltration comprenant deux organes de contrôle d'une quantité de liquide, tels que deux débitmètres, disposés sur un circuit de liquide de dialyse respectivement en amont et en aval d'un hémodialyseur, les deux débitmètres étant reliés en série directement de façon répétitive par une ligne de dérivation à l'hémodialyseur pour être soumis à un étalonnage. Dans cet agencement, selon encore une autre caractéristique de l'invention, la purge de la première chambre du filtre est commandée lors de l'étalonnage des débitmètres suivant l'instant où le niveau d'encrassement mesuré atteint le seuil d'encrassement prédéterminé.

D'autres caractéristiques et avantages de l'invention apparaîtront à la lecture de la description qui va suivre.

On se reportera aux dessins annexés sur lesquels :
La figure 1 est un schéma d'un dispositif de dialyse comprenant un système de filtration de liquide de dialyse selon l'invention ; et
La figure 2 est un schéma d'un dispositif d'hémodiafiltration comprenant un système de filtration de liquide de traitement selon l'invention.

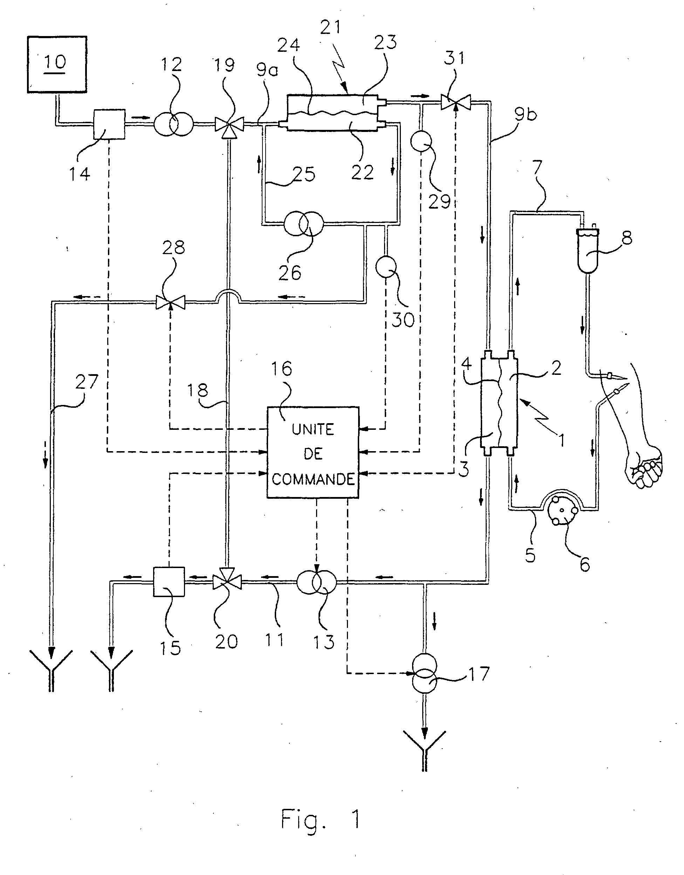

Le dispositif de traitement de sang par circulation extracorporelle représenté sur la figure 1 est propre à effectuer un traitement de dialyse. Ce dispositif comprend un hémodialyseur 1 ayant deux compartiments 2, 3 séparés par une membrane semi-perméable 4, un premier compartiment 2 étant relié à un circuit de circulation extracorporelle de sang, un second compartiment 3 étant relié à un circuit de circulation de liquide de dialyse. De façon classique, tous les composants du dispositif qui va être décrit ci-dessous, à l'exception du circuit de circulation de sang et de l'hémodialyseur, sont agencés au sein d'une machine dite de dialyse.

Le circuit de circulation de sang comprend une canalisation de prélèvement 5 connectée à une entrée du premier compartiment 2, sur laquelle est disposée une pompe 6, et une canalisation de restitution 7 connectée à une sortie du premier compartiment 2, sur laquelle est monté un piège à bulles 8.

Le circuit de liquide de dialyse comprend une canalisation d'alimentation (9a, 9b) en liquide de dialyse frais, reliant une source de liquide de dialyse 10 à une entrée du second compartiment 3 de l'hémodialyseur 1, et une canalisation d'évacuation 11 de liquide usé reliant une sortie du second compartiment 3 de l'hémodialyseur 1 à l'égout.

La source de liquide de dialyse est, par exemple, un générateur de liquide de dialyse 10 au moyen duquel de l'eau est réchauffée, dégazée puis mélangée, dans des proportions déterminées, à des solutions concentrées contenant les principaux électrolytes du sang. Le liquide de dialyse produit n'est ni stérile, ni apyrogène.

Le circuit de liquide de dialyse comprend des moyens de circulation de liquide de dialyse constitués par une première pompe 12 disposée sur la canalisation d'alimentation (9a, 9b) et une deuxième pompe 13 disposée sur la canalisation d'évacuation 11.

Le dispositif comprend aussi un système de maîtrise volumétrique de l'ultrafiltration comprenant un premier moyen de contrôle d'une quantité de liquide de dialyse, tel qu'un premier débitmètre 14, disposé sur la canalisation d'alimentation (9a, 9b) et un second moyen de contrôle d'une quantité de liquide de dialyse, tel qu'un second débitmètre 15, disposé sur la canalisation d'évacuation 11 en aval de la deuxième pompe de circulation 13. Les mesures effectuées par les deux débitmètres sont comparées par une unité de calcul et de commande 16 qui pilote la deuxième pompe 13 de circulation de liquide de dialyse de façon que les débits mesurés par les deux débitmètres soient identiques. Le système de maîtrise de l'ultrafiltration comprend en outre une pompe d'ultrafiltration 17 connectée à la canalisation d'évacuation en amont de la seconde pompe 13. Grâce à l'asservissement de la seconde pompe 13 décrit plus haut, la quantité de liquide extraite du circuit de liquide de dialyse par la pompe d'ultrafiltration 17 correspond exactement à la quantité d'eau plasmatique qui passe du sang dans le liquide de dialyse par ultrafiltration au travers de la membrane 4 sous l'effet de la dépression relative créée dans le circuit de liquide de dialyse par la pompe d'ultrafiltration 17. Une canalisation de dérivation 18 relie la canalisation d'alimentation (9a, 9b) à la canalisation d'évacuation 11 auxquelles elle est connectée, par l'intermédiaire de deux vannes trois voies 19, 20, respectivement en aval du premier débitmètre 14 et en amont du second débitmètre 15. Cette ligne de dérivation 18 permet de disposer les débitmètres 14, 15 directement en série pour un étalonnage programmé à intervalles réguliers.

Le dispositif comprend en outre un filtre 21 pour filtrer le liquide de dialyse produit par le générateur de liquide de dialyse 10. Le filtre 21 a une première et une seconde chambres 22, 23 séparées par une membrane de filtration 24, la première chambre 22 ayant une entrée connectée à une première portion 9a de la canalisation d'alimentation et la seconde chambre 23 ayant une sortie connectée à une seconde portion 9b de la canalisation d'alimentation, sur laquelle est disposé une vanne 31.

Conformément à l'invention, une canalisation de bouclage 25, sur laquelle est disposée une pompe de balayage 26, relie une sortie de la première chambre 22 du filtre 21 à l'entrée de cette première chambre. Une canalisation de purge 27 de la première chambre 22 du filtre 21 , sur laquelle est disposé un organe de contrôle de débit tel qu'une vanne 28, est connectée à la canalisation de bouclage 25 entre la sortie de la première chambre 22 du filtre 21 et la pompe 26. La canalisation de purge 27 est reliée à l'égout par une sortie de la machine de dialyse distincte de la sortie qui forme l'extrémité de la canalisation d'évacuation 11 du liquide usé.

Deux capteurs de pression 29, 30 sont disposés respectivement sur la seconde portion 9b de la canalisation d'alimentation et sur la canalisation de bouclage 25, en sortie de la première et de la deuxième chambres 22, 23 du filtre 21, pour mesurer la pression dans ces chambres. Les informations délivrées par les capteurs de pression 29, 30 sont fournies à l'unité de commande et de calcul 16 qui peut calculer la pression transmembranaire dans le filtre 21 et commander le fonctionnement du dispositif en fonction des valeurs mesurées et calculées des pressions dans le filtre 21, comme cela va être expliqué dans ce qui suit.

Lorsque les étapes préparatoires au traitement sont achevées, c'est-à-dire le rinçage et le remplissage initiaux du circuit de liquide de dialyse 9a, 9b, 11, de l'hémodialyseur 1, et du circuit sang 5, 7, 8 et la connexion du circuit sang au circuit vasculaire du patient, le liquide de dialyse produit par le générateur 10 de liquide de dialyse est mis en circulation dans le circuit de liquide de dialyse au moyen des pompes 12 et 13, et le sang du patient est mis en circulation dans le circuit sang au moyen de la pompe 6 (la vanne 31 est alors ouverte et les vannes 19, 20 sont disposées de façon à permettre la circulation dans le canalisation d'alimentation 9a, 9b et dans la canalisation d'évacuation 11).

En outre, la vanne 28 de la canalisation de purge 27 est fermée et la pompe de balayage 26 tourne à une vitesse prédéterminée de sorte que du liquide non filtré provenant du générateur circule en permanence dans la première chambre 22 du filtre 21 et balaye la membrane 24, ce qui a pour effet de s'opposer à son encrassement en maintenant en suspension les impuretés arrêtées par la membrane du filtre.

De façon classique, les débitmètres 14 et 15 sont étalonnés à intervalles de temps réguliers. Lors de ces étalonnages successifs, les vannes 19 et 20 sont disposées de façon que le liquide de dialyse circule dans la canalisation de dérivation 18, et la première pompe de circulation 12 et la pompe d'ultrafiltration 17 tournent à leur valeur de consigne tandis que la deuxième pompe de circulation 13 est stoppée.

Selon l'invention, la purge de la première chambre 22 du filtre 21, destinée à éliminer les impuretés arrêtées par la membrane 24, est commandée lorsqu'un niveau d'encrassement prédéterminé de la membrane est détecté. Plus précisément, l'unité de commande 16 compare soit en continu, soit à intervalles de temps réguliers, la valeur instantanée de la pression transmembranaire dans le filtre 21, calculée à partir des informations délivrées par les capteurs de pression 29, 30, à une valeur de référence calculée en début de séance. Lorsque la valeur instantanée excède la valeur de référence d'une quantité prédéterminée, une purge de la première chambre 22 du filtre 21 est commandée lors de la phase d'étalonnage des débitmètres 14, 15 subséquente. La vanne 31 est alors fermée et la vanne de purge 28 est ouverte le temps nécessaire à l'évacuation, par la canalisation de purge 27, du liquide contenu dans la première chambre 22 du filtre 21 et dans la canalisation de bouclage 25.

Selon l'invention, la fréquence des purges est calculée par l'unité de calcul et est comparée à une fréquence de référence. Si la fréquence calculée atteint ou excède la fréquence de référence, la vitesse de rotation de la pompe 26 est augmentée pour que l'efficacité du nettoyage de la membrane par balayage tangentiel soit accrue.

Selon l'invention, on mesure le vieillissement du filtre 21 en partant de l'observation qu'au cours du temps et en dépit du nettoyage continu auquel la membrane 24 est soumise, la pression transmembranaire dans le filtre augmente, à débits de liquide de dialyse égaux. L'unité de commande et de calcul 16 compare donc à intervalles de temps réguliers une pression transmembranaire de référence, correspondant par exemple à la pression transmembranaire du filtre calculée à sa première mise en service pour un débit de liquide de dialyse fixé, à la pression transmembranaire instantanée mesurée au même débit, et lorsque celle-ci s'écarte de celle-là d'une quantité prédéterminée, elle émet une alarme ou un message sur une unité d'affichage (non représentée) pour signaler à l'utilisateur que le filtre 21 doit être changé.

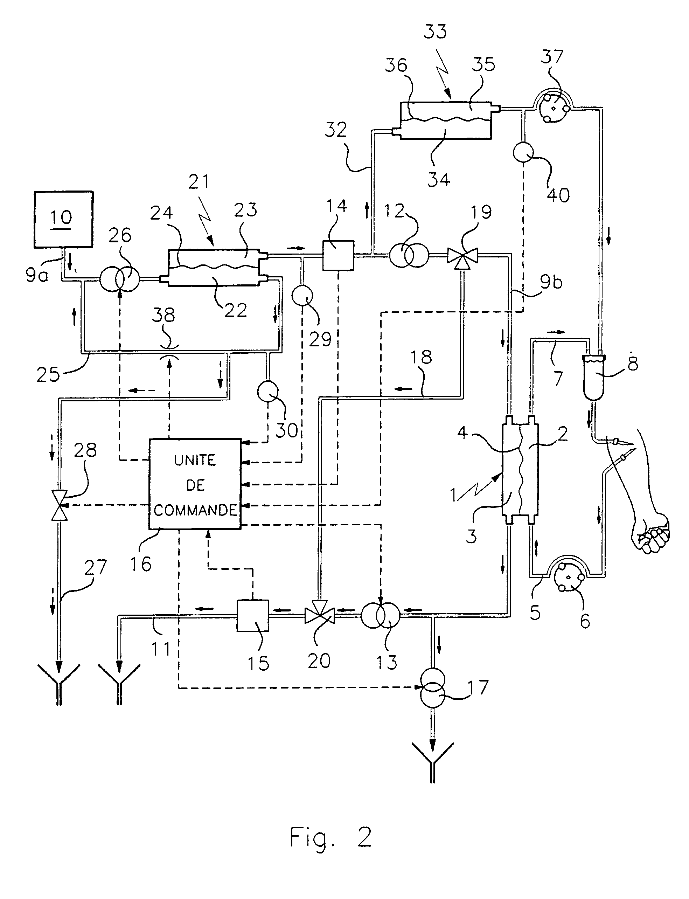

La figure 2 représente un dispositif d'hémodiafiltration qui se différencie du dispositif d'hémodialyse décrit ci-dessus par les caractéristiques suivantes (les composants de ces deux dispositifs remplissant les mêmes fonctions ont été désignés par les mêmes chiffres de référence).

Conformément à sa vocation, ce dispositif comporte des moyens pour infuser au patient un liquide de substitution destiné à compenser, généralement en partie, la quantité d'eau plasmatique soutirée du circuit vasculaire du patient par ultrafiltration dans l'hémodialyseur à haute perméabilité 1. Les moyens d'infusion comprennent une canalisation de liquide de substitution 32 ayant une première portion reliant la canalisation d'alimentation de liquide de dialyse 9a, 9b à une entrée d'une première chambre 34 d'un second filtre 33, et une seconde portion reliant une sortie d'une seconde chambre 35, séparée de la première chambre 34 par une membrane de filtration 36, au piège à bulles 8 du circuit de circulation extracorporelle de sang. Une pompe de circulation 37 de liquide de substitution est disposée sur la seconde portion de la canalisation de liquide de substitution 32. La canalisation de liquide de substitution est connectée à la canalisation d'alimentation 9a, 9b entre l'organe pour mesurer une quantité de liquide de dialyse 14 (débitmètre par exemple) et la première pompe 12 de circulation de liquide de dialyse.

Dans ce dispositif, le premier filtre 21 n'est pas connecté à la canalisation d'alimentation 9a, 9b de liquide de dialyse à l'intérieur du système de maîtrise volumétrique d'ultrafiltration (débitmètres 14, 15, portion du circuit de liquide de dialyse s'étendant entre les débitmètres et pompe d'ultrafiltration 17) mais à l'extérieur de ce système, en amont du premier débitmètre 14. Comme cela a été mentionné plus haut, grâce à cette disposition le liquide circulant dans le premier débitmètre 14 est un liquide filtré, ce qui prévient l'encrassement et, le cas échéant, le blocage accidentel, de cet organe de mesure sensible.

Autre différence avec le dispositif représenté sur la figure 1, la pompe de balayage 26 est disposée immédiatement en amont de la première chambre 22 du premier filtre 21, sur une portion de canalisation commune à la canalisation d'alimentation 9a et à la canalisation de bouclage 25. En fonctionnement, la pompe de balayage 26 est réglée à un débit supérieur à la somme des débits de la pompe de circulation 12 et de la pompe de liquide de substitution 37, de façon qu'un débit de recirculation déterminé s'établisse dans la canalisation de bouclage 25. Un organe de rétreint 38, éventuellement réglable, est disposé sur la canalisation de bouclage 25, grâce auquel il est possible d'obtenir une pression suffisante dans le circuit de liquide de dialyse en amont de la pompe de circulation 12 pour que la pression dans la seconde chambre 35 du second filtre 33, telle que mesurée par un capteur de pression 40, soit toujours positive ou au minimum nulle, quel que soit le débit de la pompe de liquide de substitution 37. L'organe de rétreint 38 peut être constitué par une portion calibrée de la canalisation de bouclage 25.

Le fonctionnement de ce deuxième dispositif est le suivant, pour ce qu'il diffère de celui du dispositif représenté sur la figure 1.

Les diverses pompes de l'installation tournant aux débits prescrits par l'opérateur ou programmés par défaut ou calculés, soit en début soit en cours de séance, l'unité de calcul et de commande 16 compare, soit en continu, soit à intervalles de temps réguliers, la pression mesurée au moyen du capteur 29 dans la seconde chambre 23 du filtre 21 à une pression de consigne et elle pilote la pompe de balayage 26 de façon que la pression instantanée tende vers la pression de consigne. La pression de consigne est choisie pour que la pression dans la seconde chambre 35 du second filtre 33, telle que mesurée par le capteur de pression 40, soit toujours positive ou au minimum nulle, compte tenu du débit de liquide de substitution imposé à la pompe 37.

Pour mesurer le degré d'encrassement de la membrane du filtre 21, deux procédés sont possibles. Soit, comme décrit ci-dessus, la pression transmembranaire dans le filtre 21 est calculée grâce aux données fournies par les capteurs de pression 29, 30 et est comparée à une pression transmembranaire de référence mise en mémoire (par exemple, la pression transmembranaire mesurée en début de séance). Soit, la vitesse de rotation de la pompe de balayage 26 est comparée à une vitesse de référence (par exemple, la vitesse de rotation mesurée en début de séance ou à chaque mise en service du filtre (21) et mise en mémoire dans l'unité de commande 16). Comme la pression est maintenue constante dans la deuxième chambre 23 du filtre 21 par le réglage de la vitesse de rotation de la pompe de balayage 26, si la membrane 24 s'encrasse, la vitesse de rotation de la pompe sera augmentée d'autant. Lorsque la valeur mesurée du paramètre considéré (pression transmembranaire ou vitesse de rotation de la pompe de balayage) atteint la valeur de référence correspondante, l'unité de commande 16 commande l'ouverture de la vanne de purge 28 le temps nécessaire à l'évacuation du liquide contenu dans la première chambre 23 du filtre 21 et dans la canalisation de bouclage 25.

L'invention n'est pas limitée aux modes de réalisation spécifiques qui viennent d'être décrits et elle est susceptible de variantes. En particulier, bien que cette variante puisse être considérée comme moins avantageuse, la canalisation de purge 27 du dispositif de la figure 1 pourrait être connectée à la canalisation d'évacuation 11 de liquide usé à l'intérieur du système de maîtrise volumétrique de l'ultrafiltration, c'est-à-dire en amont des seconds moyens de mesure 15 d'une quantité de liquide usé.

Par ailleurs, l'organe de contrôle de débit 28 disposé sur la canalisation de purge 27 pourrait être constitué par une pompe. Dans le dispositif de la figure 2, cette pompe pourrait tourner de façon continue de façon que les impuretés retenues par la membrane soient éliminées au fur et à mesure.

## Revendications

1. Dispositif de traitement de sang par circulation extracorporelle comprenant un circuit de liquide de dialyse ayant :
- une canalisation d'alimentation (9a, 9b) sur laquelle est disposé un filtre (21) ayant une première et une seconde chambres (22, 23) séparées par une membrane de filtration (24), la canalisation d'alimentation ayant une première portion (9a) reliant une source de liquide de dialyse (10) à une entrée de la première chambre (22); et une seconde portion (9b) ayant une extrémité connectée à une sortie de la seconde chambre (23) du filtre (21) et une autre extrémité connectable à une entrée d'un compartiment (3) d'un échangeur (1 ) à membrane semiperméable ;
- une canalisation d'évacuation (11) ayant une extrémité connectable à une sortie du compartiment (3) de l'échangeur (1) ; et
- une canalisation de purge (27), sur laquelle est disposé un organe de contrôle de débit (28), connectée à une sortie de la première chambre (22) du filtre (21),
une canalisation de bouclage (25) reliant l'entrée de la première chambre (22) du filtre (21) à la sortie de la première chambre (22), sur laquelle est disposée une pompe de balayage (26) pour faire circuler du liquide dans la première chambre (22) du filtre (21) et provoquer un nettoyage de la membrane (24) par balayage tangentiel, **caractérisé en ce qu'**il comporte des moyens (29) pour mesurer la pression dans la seconde chambre (23) du filtre (21) et des moyens (16) pour commander la pompe de balayage (26) en fonction de la pression dans là seconde chambre du filtre (21) pour maintenir une pression sensiblement constante dans la seconde chambre (23) du filtre (21).

2. Dispositif selon la revendication 1, **caractérisé en ce qu'**il comprend des moyens (29, 30 ; 26) pour détecter un encrassement de la membrane (24) du filtre (21) et des moyens de commande (16) pour commander l'organe de contrôle de débit (28) en fonction d'un seuil d'encrassement prédéterminé de la membrane (24) de façon à provoquer une purge de la première chambre (22) du filtre (21).

3. Dispositif selon la revendication 2, **caractérisé en ce que** les moyens pour détecter un encrassement de la membrane du filtre (21) comportent :
- des moyens (29, 30) pour mesurer la pression transmembranaire entre la première et la seconde chambre (22, 23) du filtre (21), et
- des moyens (16) pour comparer la pression transmembranaire mesurée à une pression transmembranaire de référence, les moyens de commande (16) agissant sur l'organe de contrôle de débit (28) pour provoquer une purge de la première chambre (22) du filtre (21) lorsque la pression transmembranaire mesurée atteint la pression transmembranaire de référence.

4. Dispositif selon la revendication 3, **caractérisé en ce que** la pression transmembranaire de référence est une pression transmembranaire mesurée à chaque mise en service du filtre (21).

5. Dispositif selon une des revendications 1 à 4, **caractérisé en ce que** la pompe de balayage (26) est disposée sur une portion de canalisation commune à la canalisation de bouclage (25) et à la première portion (9a) de la canalisation d'alimentation, cette portion commune étant connectée à l'entrée de la première chambre (22) du filtre (21).

6. Dispositif selon les revendications 2, **caractérisé en ce que** les moyens pour détecter un encrassement de la membrane (24) du filtre (21) comportent :
- des moyens pour mesurer la vitesse de rotation de la pompe de balayage (26), et
- des moyens (16) pour comparer la vitesse de rotation mesurée à une vitesse de rotation de référence, les moyens de commande (16) agissant sur l'organe de contrôle de débit (28) pour provoquer une purge de la première chambre (22) du filtre (21) lorsque la vitesse de rotation mesurée atteint la vitesse de rotation de référence.

7. Dispositif selon la revendication 6, **caractérisé en ce que** la vitesse de rotation de référence est une vitesse mesurée à chaque mise en service du filtre (21).

8. Dispositif selon une des revendications 2 à 7, **caractérisé en ce qu'**il comporte des moyens pour calculer la fréquence des purges de la première chambre (22) du filtre (21).

9. Dispositif selon la revendication 8, **caractérisé en ce qu'**il comporte des moyens (16) pour comparer la fréquence des purges calculée à une fréquence de référence, les moyens de commande (16) étant en outre prévus pour augmenter le débit de la pompe de balayage (26) lorsque la fréquence des purges calculée atteint la fréquence de référence.

10. Dispositif selon la revendication 3, **caractérisé en ce qu'**il comporte des moyens (16) pour mémoriser une pression transmembranaire initiale à la première mise en service du filtre (21) et des moyens (16) pour comparer la pression transmembranaire initiale à la pression transmembranaire mesurée.

11. Dispositif selon la revendication 10, **caractérisé en ce qu'**il comporte en outre des moyens d'alarme pour émettre une alarme lorsque la pression transmembranaire mesurée s'écarte d'une quantité prédéterminée de la pression transmembranaire initiale.

12. Dispositif selon une des revendications 1 à 11, **caractérisé en ce qu'**il comporte des moyens de contrôle (14) d'une quantité de liquide de dialyse, tels qu'un débitmètre, disposés sur la canalisation d'alimentation (9a, 9b) et **en ce que** le filtre (21) est placé sur la canalisation d'alimentation (9a, 9b) entre la source (10) de liquide de dialyse et les moyens de contrôle (14) d'une quantité de liquide de dialyse.

13. Dispositif selon la revendication 12, **caractérisé en ce qu'**il comporte en outre :
- un second filtre (33) ayant une première et une seconde chambres (34, 35) séparées par une membrane de filtration (36) ;
- une canalisation de liquide de substitution (32) ayant :
· une première portion pour relier une entrée de la première chambre (34) du second filtre (33) à la canalisation (9a, 9b) d'alimentation en liquide de dialyse entre les moyens (14) de contrôle de la quantité de liquide de dialyse et une pompe de circulation (12) de liquide de dialyse disposée sur la canalisation d'alimentation (9a, 9b) ; et
· une deuxième portion, sur laquelle est disposée une pompe de liquide de substitution (37), pour relier une sortie de la seconde chambre (35) du second filtre (33) à un circuit de circulation extracorporelle de sang ;
- un organe de contrôle (38) de débit de liquide disposé sur la canalisation de bouclage (25) pour ajuster la pression dans la canalisation d'alimentation (9b) en amont de la pompe de circulation (12) de façon que la pression dans la seconde chambre (35) du second filtre (33) soit toujours positive ou au minimum nulle, quel que soit le débit de la pompe de liquide de substitution (37).

14. Procédé de nettoyage d'un filtre (21) ayant deux chambres séparées (22, 23) par une membrane de filtration (24), une première chambre (22) étant reliée à une source (10) de liquide de traitement, une seconde chambre (23) ayant une sortie pour du liquide de traitement filtré, permettant de faire recirculer de façon continue du liquide de traitement dans la première chambre (22) pour provoquer un balayage tangentiel de la membrane (24) du filtre (21) et contrarier l'encrassement de la membrane (24) par les substances arrêtées par la membrane, **caractérisé en ce que** la pression est maintenue sensiblement constante dans la seconde chambre du filtre (21).

15. Procédé selon la revendication 14, **caractérisé en ce qu'**il comprend les étapes de :
- mesurer un niveau d'encrassement du filtre (21) ;
- comparer le niveau d'encrassement mesuré à un seuil d'encrassement prédéterminé ; et
- purger la première chambre (22) du filtre (21) lorsque le niveau d'encrassement mesuré atteint le seuil d'encrassement prédéterminé.

16. Procédé selon la revendication 15, **caractérisé en ce que** l'étape de mesurer un niveau d'encrassement du filtre (21) comprend les étapes de :
- mesurer une pression transmembranaire du filtre (21) ; et
- comparer la pression transmembranaire mesurée à une pression transmembranaire de référence.

17. Procédé selon la revendication 16, **caractérisé en ce que** la pression transmembranaire de référence est une pression transmembranaire mesurée à chaque mise en service du filtre (21).

18. Procédé selon les revendications 15, **caractérisé en ce que** l'étape de mesurer un niveau d'encrassement du filtre (21) comprend les étapes de :
- mesurer la vitesse de rotation d'une pompe (26) utilisée pour faire recirculer le liquide de traitement dans la première chambre (22) du filtre (21) ;
- comparer la vitesse de rotation mesurée à une vitesse de rotation de référence.

19. Procédé selon la revendication 18, **caractérisé en ce que** la vitesse de rotation de référence est une vitesse mesurée à chaque mise en service du filtre (21).

20. Procédé selon une des revendications 14 à 19, **caractérisé en ce qu'**il consiste en outre à comparer la fréquence effective des purges de la première chambre (22) du filtre (21) à une fréquence de référence et à augmenter le débit de recirculation dans la première chambre (22) du filtre (21) lorsque la fréquence effective atteint la fréquence de référence.

21. Procédé selon la revendication 14, adapté à un filtre (21) disposé dans un système de maîtrise volumétrique d'ultrafiltration d'une machine d'hémodialyse/hémofiltration, le système de maîtrise d'ultrafiltration comprenant deux organes de contrôle d'une quantité de liquide, tels que deux débitmètres (14, 15), disposés sur un circuit de liquide de dialyse (9a, 9b, 11) respectivement en amont et en aval d'un hémodialyseur (1), les deux débitmètres (14, 15) étant reliés en série directement de façon répétitive par une ligne de dérivation (18) à l'hémodialyseur (1) pour être soumis à un étalonnage, **caractérisé en ce que** la purge de la première chambre (22) du filtre (21) est commandée lors d'un étalonnage des débitmètres (14, 15) suivant l'instant où le niveau d'encrassement mesuré atteint le seuil d'encrassement prédéterminé.

## Claims

1. Device for treatment of blood by extracorporeal circulation comprising a dialysis liquid circuit having:
- a supply tube (9a, 9b) on which is arranged a filter (21) having a first chamber and a second chamber (22, 23) which are separated by a filtering membrane (24), the supply tube having a first portion (9a) connecting a dialysis liquid source (10) to an inlet of the first chamber (22), and a second portion (9b) having an end connected to an outlet of the second chamber (23) of the filter (21) and another end which can be connected to an inlet of a compartment (3) of an exchanger (1) with semipermeable membrane;
- a discharge tube (11) having an end which can be connected to an outlet of the compartment (3) of the exchanger (1 ); and
- a purge tube (27) on which a flow-control element (28) is arranged and which is connected to an outlet of the first chamber (22) of the filter (21),
- a feedback tube (25) connecting the inlet of the first chamber (22) of the filter (21) to the outlet of the first chamber (22), and on which is arranged a flushing pump (26) for circulating liquid in the first chamber (22) of the filter (21) and causing cleaning of the membrane (24) by tangential flushing,
**characterized in that** it includes means (29) for measuring the pressure in the second chamber (23) of the filter (21) and means (16) for controlling the flushing pump (26) as a function of the pressure in the second chamber of the filter (21) in order to keep a substantially constant pressure in the second chamber (23) of the filter (21).

2. Device according to Claim 1, **characterized in that** it comprises means (29, 30; 26) for detecting a clogging of the membrane (24) of the filter (21) and control means (16) for controlling the flow-control element (28) as a function of a predetermined clogging threshold of the membrane (24) so as to cause purging of the first chamber (22) of the filter (21).

3. Device according to Claim 2, **characterized in that** the means for detecting clogging of the membrane of the filter (21) include:
- means (29, 30) for measuring the transmembrane pressure between the first chamber and the second chamber (22, 23) of the filter (21), and
- means (16) for comparing the transmembrane pressure measured with a reference transmembrane pressure, the control means (16) acting on the flow-control element (28) in order to cause purging of the first chamber (22) of the filter (21) when the transmembrane pressure measured reaches the reference transmembrane pressure.

4. Device according to Claim 3, **characterized in that** the reference transmembrane pressure is a transmembrane pressure measured each time the filter (21) is set in operation.

5. Device according to one of Claims 1 to 4, **characterized in that** the flushing pump (26) is arranged on a tube portion common to the feedback tube (25) and at the first portion (9a) of the supply tube, this common portion being connected to the inlet of the first chamber (22) of the filter (21).

6. Device according to Claim 2, **characterized in that** the means for detecting clogging of the membrane (24) of the filter (21) include:
- means for measuring the speed of rotation of the flushing pump (26), and
- means (16) for comparing the speed of rotation measured with a reference speed of rotation, the control means (16) acting on the flow-control element (28) in order to cause purging of the first chamber (22) of the filter (21) when the speed of rotation measured reaches the reference speed of rotation.

7. Device according to Claim 6, **characterized in that** the reference speed of rotation is a speed measured each time the filter (21) is set in operation.

8. Device according to one of Claims 2 to 7, **characterized in that** it includes means for calculating the frequency of the purges of the first chamber (22) of the filter (21).

9. Device according to Claim 8, **characterized in that** it includes means (16) for comparing the calculated frequency of the purges with a reference frequency, the control means (16) being furthermore designed to increase the flow rate of the flushing pump (26) when the calculated frequency of the purges reaches the reference frequency.

10. Device according to Claim 3, **characterized in that** it includes means (16) for storing in memory an initial transmembrane pressure when the filter (21) is set in operation for the first time and means (16) for comparing the initial transmembrane pressure with the transmembrane pressure measured.

11. Device according to Claim 10, **characterized in that** it furthermore includes alarm means for emitting an alarm when the transmembrane pressure measured deviates by a predetermined quantity from the initial transmembrane pressure.

12. Device according to one of Claims 1 to 11, **characterized in that** it includes means (14), such as a flow meter, for monitoring a quantity of dialysis liquid, which means are arranged on the supply tube (9a, 9b), and **in that** the filter (21) is placed on the supply tube (9a, 9b) between the dialysis liquid source (10) and the means (14) for monitoring a quantity of dialysis liquid.

13. Device according to Claim 12, **characterized in that** it furthermore includes:
- a second filter (33) having a first chamber and a second chamber (34, 35) which are separated by a filtering membrane (36);
- a substitution liquid tube (32) having:
• a first portion for connecting an inlet of the first chamber (34) of the second filter (33) to the dialysis-liquid supply tube (9a, 9b) between the means (14) for monitoring the quantity of dialysis liquid and a pump (12) for circulating dialysis liquid, arranged on the supply tube (9a, 9b); and
• a second portion, on which is arranged a substitution-liquid pump (37) for connecting an outlet of the second chamber (35) of the second filter (33) to an extracorporeal blood circulation circuit;
- a liquid flow-control element (38) arranged on the feedback tube (25) for adjusting the pressure in the supply tube (9b) upstream of the circulation pump (12) so that the pressure in the second chamber (35) of the second filter (33) is always positive or at least zero, whatever the flow rate of the substitution-liquid pump (37).

14. Method for cleaning a filter (21) having two chambers (22, 23) separated by a filtering membrane (24), a first chamber (22) being connected to a treatment liquid source (10), a second chamber (23) having an outlet for the filtered treatment liquid, allowing to continuously recirculate treatment liquid into the first chamber (22) in order to cause tangential flushing of the membrane (24) of the filter (21) and to prevent clogging of the membrane (24) by the substances stopped by the membrane, **characterized in that** the pressure is kept substantially constant in the second chamber of the filter (21).

15. Method according to Claim 14, **characterized in that** it comprises the steps of:
- measuring a clogging level of the filter (21 );
- comparing the clogging level measured with a predetermined clogging threshold; and
- purging the first chamber (22) of the filter (21) when the clogging level measured reaches the predetermined clogging threshold.

16. Method according to Claim 15, **characterized in that** the step of measuring a clogging level of the filter (21) comprises the steps of:
- measuring a transmembrane pressure of the filter (21); and
- comparing the transmembrane pressure measured with a reference transmembrane pressure.

17. Method according to Claim 16, **characterized in that** the reference transmembrane pressure is a transmembrane pressure measured each time the filter (21) is set in operation.

18. Method according to Claim 15, **characterized in that** the step of measuring a clogging level of the filter (21) comprises the steps of:
- measuring the speed of rotation of a pump (26) used for recirculating the treatment liquid into the first chamber (22) of the filter (21);
- comparing the speed of rotation measured with a reference speed of rotation.

19. Method according to Claim 18, **characterized in that** the reference speed of rotation is a speed measured each time the filter (21) is set in operation.

20. Method according to one of Claims 14 to 19, **characterized in that** it furthermore consists in comparing the actual frequency of the purges of the first chamber (22) of the filter (21) with a reference frequency and in increasing the rate of recirculation into the first chamber (22) of the filter (21) when the actual frequency reaches the reference frequency.

21. Method according to Claim 14, adapted to a filter (21) arranged in a volumetric ultrafiltration control system of a haemodialysis/haemofiltration machine, the ultrafiltration control system comprising two elements for monitoring a quantity of liquid, such as two flow meters (14, 15), arranged on a dialysis liquid circuit (9a, 9b, 11 ) respectively upstream and downstream of a haemodialyser (1 ), the two flow meters (14, 15) being directly connected in series in repetitive fashion by a branch line (18) to the haemodialyser (1) in order to be subjected to calibration, **characterized in that** purging of the first chamber (22) of the filter (21) is ordered during calibration of the flow meters (14, 15) following the instant when the clogging level measured reaches the predetermined clogging threshold.

## Patentansprüche

1. Vorrichtung zur Behandlung von Blut durch einen extrakorporalen Kreislauf mit einer Dialyseflüssigkeitsleitung, die Folgendes aufweist:
- eine Versorgungsleitung (9a, 9b) in der ein Filter (21) mit einer ersten und einer zweiten Kammer (22, 23) angeordnet ist, die durch eine Filtermembran (24) getrennt sind, wobei die Versorgungsleitung einen ersten Abschnitt (9a), der eine Quelle für Dialyseflüssigkeit (10) mit einem Eingang der ersten Kammer (22) verbindet, und einen zweiten Abschnitt (9b) aufweist, der ein an einen Ausgang der zweiten Kammer (23) des Filters (21) angeschlossenes Ende und ein anderes, an einen Eingang eines Teilraums (3) eines Tauschers (1) mit einer halbdurchlässigen Membran anschließbares Ende aufweist,
- eine Abflussleitung (11) mit einem an einen Eingang des Teilraums (3) des Tauschers (1) anschliessbaren Ende, und
- einer Entleerungsleitung (27), in der ein Durchflusssteuerungsorgan (28) angeordnet ist, das an einen Ausgang der ersten Kammer (22) des Filters (21) angeschlossen ist,
- eine Leitungsschleife (25), die den Eingang der ersten Kammer (22) des Filters (21) mit dem Ausgang der ersten Kammer (22) verbindet und in der eine Spülpumpe (26) angeordnet ist, um Flüssigkeit in der ersten Kammer (22) des Filters (21) zirkulieren zu lassen und eine Reinigung der Membran (24) durch tangentiales Überstreichen zu bewirken,
**dadurch gekennzeichnet, dass** sie Mittel (29) umfasst, um den Druck in der zweiten Kammer (23) des Filters (21) zu messen, und Mittel (16) um die Spülpumpe (26) in Anhängigkeit von dem Druck in der zweiten Kammer des Filters (21) zu betätigen, um einen im Wesentlichen konstanten Druck in der zweiten Kammer (23) des Filters (21) aufrechtzuerhalten.

2. Vorrichtung nach Anspruch 1, **dadurch gekennzeichnet, dass** sie Mittel (29, 30; 26) umfasst, um eine Verschmutzung der Membran (24) des Filters (21) zu erfassen und Steuermittel (16), um das Durchflusssteuerungsorgan (28) in Abhängigkeit von einem vorbestimmten Verschmutzungsgrenzwert für die Membran (24) zu steuern, um eine Entleerung der ersten Kammer (22) des Filters (21) hervorzurufen.

3. Vorrichtung nach Anspruch 2, **dadurch gekennzeichnet, dass** die Mittel zur Erfassung einer Verschmutzung der Membran des Filters (21) Folgendes umfassen:
- Mittel (29, 30) zum Messen des Drucks über die Membran zwischen der ersten Kammer und der zweiten Kammer (22, 23) des Filters (21), und
- Mittel (16) zum Vergleichen des gemessenen Drucks über der Membran mit einem Referenzdruck über der Membran, wobei die Steuermittel (16) auf das Durchflusssteuerungsorgan (28) einwirken, um eine Entleerung der ersten Kammer (22) des Filters (21) hervorzurufen, wenn der gemessene Druck über die Membran den Referenzdruck über die Membran erreicht.

4. Vorrichtung nach Anspruch 3, **dadurch gekennzeichnet, dass** der Referenzdruck über die Membran der bei jeder Inbetriebnahme des Filters (21) gemessene Druck ist.

5. Vorrichtung nach einem der Ansprüche 1 bis 4, **dadurch gekennzeichnet, dass** die Spülpumpe (26) auf einem Leitungsabschnitt angeordnet ist, der der Leitungsschleife (25) und dem ersten Abschnitt (9a) der Versorgungsleitung gemeinsam ist, wobei dieser gemeinsame Abschnitt mit dem Eingang der ersten Kammer (22) des Filters (21) verbunden ist.

6. Vorrichtung nach Anspruch 2, **dadurch gekennzeichnet, dass** die Mittel zur Erfassung einer Verschmutzung der Membran (24) des Filters (21) umfassen:
- Mittel zum Messen der Drehgeschwindigkeit der Spülpumpe (26), und
- Mittel (16) zum Vergleichen der gemessenen Drehgeschwindigkeit mit einer Referenzdrehgeschwindigkeit, wobei die Steuermittel (16) auf das Durchflusssteuerungsorgan (28) einwirken, um eine Entleerung der ersten Kammer (22) des Filters (21) hervorzurufen, wenn die gemessene Drehgeschwindigkeit die Referenzdrehgeschwindigkeit erreicht.

7. Vorrichtung nach Anspruch 6, **dadurch gekennzeichnet, dass** die Referenzdrehgeschwindigkeit die bei jeder Inbetriebnahme des Filters (21) gemessene Geschwindigkeit ist.

8. Vorrichtung nach einem der Ansprüche 2 bis 7, **dadurch gekennzeichnet, dass** sie Mittel zum Berechnen der Häufigkeit von Entleerungen der ersten Kammer (22) des Filters (21) umfasst.

9. Vorrichtung nach Anspruch 8, **dadurch gekennzeichnet, dass** sie Mittel (16) zum Vergleichen der berechneten Häufigkeit von Entleerungen mit einer Referenzhäufigkeit umfasst, wobei die Steuermittel (16) außerdem dafür vorgesehen sind, den Durchsatz der Spülpumpe (26) zu erhöhen, wenn die berechnete Häufigkeit von Entleerungen die Referenzhäufigkeit erreicht.

10. Vorrichtung nach Anspruch 3, **dadurch gekennzeichnet, dass** sie Mittel (16) zum Speichern eines anfänglichen Drucks über die Membran bei der ersten Inbetriebnahme des Filters (21) und Mittel (16) zum Vergleichen des anfänglichen Drucks über die Membran mit dem gemessenen Druck über die Membran umfasst .

11. Vorrichtung nach Anspruch 10, **dadurch gekennzeichnet, dass** sie außerdem Warnmittel umfasst, um eine Warnung auszugeben, wenn der gemessene Druck über die Membran um einen vorbestimmten Betrag von dem anfänglichen Druck über die Membran abweicht.

12. Vorrichtung nach einem der Ansprüche 1 bis 11, **dadurch gekennzeichnet, dass** sie Kontrollmittel (14) für eine Dialyseflüssigkeitsmenge umfasst, wie beispielsweise einen Durchflussmesser, die in der Versorgungsleitung (9a, 9b) angeordnet sind, und dass das Filter (21) in der Versorgungsleitung (9a, 9b) zwischen der Quelle (10) für Dialyseflüssigkeit und den Kontrollmitteln (14) für eine Dialyseflüssigkeitsmenge angeordnet ist.

13. Vorrichtung nach Anspruch 12, **dadurch gekennzeichnet, dass** sie außerdem umfasst:
- ein zweites Filter (33), das eine erste und eine zweite Kammer (34, 35) aufweist, die durch eine Filtermembran (36) getrennt sind,
- eine Substitutionsflüssigkeitsleitung (32) mit:
· einem ersten Abschnitt zum Verbinden eines Eingangs der ersten Kammer (34) des zweiten Filters (33) mit der Versorgungsleitung (9a, 9b) für Dialyseflüssigkeit zwischen den Kontrollmitteln (14) für die Dialyseflüssigkeitsmenge und einer Umwälzpumpe (12) für Dialyseflüssigkeit, die in der Versorgungsleitung (9a, 9b) angeordnet ist, und
· einem zweiten Abschnitt, in dem eine Substitutionsflüssigkeitspumpe (37) angeordnet ist, um einen Ausgang der zweiten Kammer (35) des zweiten Filters (33) mit einer Leitung für den extrakorporalen Blutkreislauf zu verbinden,
- ein Flüssigkeitsdurchsatzsteuerungsorgan (38), das in der Leitungsschleife (25) angeordnet ist, um den Druck in der Versorgungsleitung (9b) stromaufwärts von der Umwälzpumpe (12) einzustellen, so dass der Druck in der zweiten Kammer (35) des zweiten Filters (33) unabhängig von dem Durchsatz der Substitutionsflüssigkeitspumpe (37) immer positiv oder im äußersten Fall Null ist.

14. Verfahren zur Reinigung eines Filters (21) mit zwei durch eine Filtermembran (24) getrennten Kammern (22, 23), wobei eine erste Kammer (22) mit einer Quelle (10) für Behandlungsflüssigkeit verbunden ist, eine zweite Kammer (23) einen Ausgang für filtrierte Behandlungsflüssigkeit aufweist, das es gestattet, kontinuierlich Behandlungsflüssigkeit in der ersten Kammer (22) zirkulieren zu lassen, um ein tangentiales Überstreichen der Membran (24) des Filters (21) hervorzurufen und der Verschmutzung der Membran (24) durch von der Membran zurückgehaltenen Substanzen entgegenzuwirken, **dadurch gekennzeichnet, dass** der Druck in der zweiten Kammer des Filters (21) im Wesentlichen konstant gehalten wird.

15. Verfahren nach Anspruch 14, **dadurch gekennzeichnet, dass** es die folgenden Schritte umfasst:
- Messen eines Verschmutzungsgrads des Filters (21) ,
- Vergleichen des gemessenen Verschmutzungsgrads mit einem vorbestimmten Verschmutzungsgrenzwert, und
- Entleeren der ersten Kammer (22) des Filters (21), wenn der gemessene Verschmutzungsgrad den vorbestimmten Verschmutzungsgrenzwert erreicht.

16. Verfahren nach Anspruch 15, **dadurch gekennzeichnet, dass** der Schritt des Messens des Verschmutzungsgrads des Filters (21) die folgenden Schritte umfasst:
- Messen eines Drucks über die Membran des Filters (21), und
- Vergleichen des gemessenen Drucks über der Membran mit einem Referenzdruck über der Membran.

17. Verfahren nach Anspruch 16, **dadurch gekennzeichnet, dass** der Referenzdruck über die Membran der bei jeder Inbetriebnahme des Filters (21) gemessene Druck ist.

18. Verfahren nach Anspruch 15, **dadurch gekennzeichnet, dass** der Schritt des Messens des Verschmutzungsgrads des Filters (21) die folgenden Schritte umfasst:
- Messen der Drehgeschwindigkeit einer Pumpe (26), die dazu verwendet wird, die Behandlungsflüssigkeit in die erste Kammer (22) des Filters (21) zurückzuleiten;
- Vergleichen der gemessenen Drehgeschwindigkeit mit einer Referenzdrehgeschwindigkeit.

19. Verfahren nach Anspruch 18, **dadurch gekennzeichnet, dass** die Referenzdrehgeschwindigkeit die bei jeder Inbetriebnahme des Filters (21) gemessene Geschwindigkeit ist.

20. Verfahren nach einem der Ansprüche 14 bis 19, **dadurch gekennzeichnet, dass** es außerdem darin besteht, die effektive Häufigkeit von Entleerungen der ersten Kammer (22) des Filters (21) mit einer Referenzhäufigkeit zu vergleichen, und den Rücklaufdurchsatz in der ersten Kammer (22) des Filters (21) zu erhöhen, wenn die effektive Häufigkeit von Entleerungen die Referenzhäufigkeit erreicht.

21. Verfahren nach Anspruch 14, das an ein Filter (21) angepasst ist, das in einem System zur volumetrischen Regelung der Ultrafiltration für ein Hämodialyse/Hämofiltrationsgerät angeordnet ist, wobei das System zur Ultrafiltrationsregelung zwei Kontrollorgane für eine Flüssigkeitsmenge, wie beispielsweise zwei Durchflussmesser (14, 15), umfasst, die in einer Dialyseflüssigkeitsleitung (9a, 9b, 11) stromaufwärts beziehungsweise stromabwärts vom einem Hämodialysator (1) angeordnet sind, wobei die zwei Durchflussmesser (14, 15) in Reihe direkt wiederholt durch eine Umgehungsleitung (18) mit dem Hämodialysator (1) verbunden werden, um einer Eichung unterzogen zu werden, **dadurch gekennzeichnet, dass** die Entleerung der ersten Kammer (22) des Filters (21) bei einer Eichung der Durchflussmesser (14, 15) entsprechend dem Zeitpunkt ausgelöst wird, zu dem der gemessene Verschmutzungsgrad den vorbestimmten Verschmutzungsgrenzwert erreicht.
